# EUROPEAN PATENT APPLICATION

(11) **EP 2 189 977 A2**
(43) Date of publication of application: **26.05.2010**
(21) Application number: 09176056.1
(22) Date of filing: 16.11.2009
(51) Int. Cl.: G10L 15/22

(54) **Voice recognition system for medical devices**

(30) Priority: 25.11.2008 US 323010
(71) Applicant: General Electric Company, Schenectady, NY 12345 (US)
(72) Inventor: Bogineni, Kiran Kimar, 560102, Karnataka (IN)
(74) Representative: Illingworth-Law, William Illingworth

(57) **Abstract**

A system for transmitting voice commands to a medical device (16) for carrying out those commands by the medical device (16). The system includes a remote control device (12) that receives the voice commands from the caregiver (10) and recognizes the caregiver (10) as being authorized to give such commands. The recognized commands are then analyzed to determine the particular command, and the signals representing that command are transmitted in digital form by a wireless protocol, such as a ZigBee wireless protocol, to a receiving module (18) incorporated into or in communication with the medical device (16). The receiving module (18) decodes the wireless protocol, identifies the particular command, and interfaces that command to the medical device (16), whereby the command effects the operation of the medical device (16), such as by silencing an alarm thereon.

## Description

### Background

The present invention relates to a control system for medical devices and, more particularly, to a voice recognition system for controlling medical devices, such as maternal and infant care devices.

There are, of course, many differing medical devices that are used in the care of patients, including, but not limited to, monitors that continually monitor a patient under care. Such monitors are often used with birthing mothers and/or neonatal infants, commonly referred to as maternal and infant care devices. In addition, there are, of course, also many times when it is necessary to communicate with the medical device, such as to input a command, change a setting, silence an alarm, and/or the like.

One of the difficulties encountered when using such medical devices, however, is that they are often located in rooms with lots of activity, e.g., a labor and delivery room or nursery, such that the need to change a control setting or provide a command input manually, e.g., by turning a knob or other input at the site of the device, can distract the caregiver from the primary responsibility of caring for the patient.

In addition, it is often desirable to maintain clean and/or sterile conditions around the patient, and the potential risks of caregivers spreading germs by physically touching the controls of medical devices are well-known. One cross-contamination solution is disclosed in U.S. Pat. No. 6,733,437 to Mackin et al., wherein an alarm silence switch of an infant care apparatus, for example, is activated by a proximity switch when a caregiver waves a hand in close proximity to a particular sensor. With such a system, the caregiver does not need to physically touch the medical device. However, while eliminating some of the problems of cross-contamination, such systems still force the caregivers to move to a position close to the medical device in order to activate the proximity switch, and it thereby removes and/or distracts the caregiver away from the patient.

Another system, using voice recognition, is also disclosed in U.S. Pat. App. No. 2008/0082339 to Li et al., which provides a device that receives voice commands to control certain operations of an oximeter. While that device communicates by a wireless protocol with the oximeter, the wireless device only sends a signal to the oximeter in order to lock or unlock the oximeter from a voice command mode, and it does not send command signals to control any actual operation of the oximeter. In addition, it incorporates the voice command into the oximeter itself, and it therefore remains inconvenient for the caregiver currently attending to a patient to operate effortlessly. Moreover, voice activation at the oximeter is subject to interference by numerous other sounds within the room.

Accordingly, while voice recognition is useful and allows a caregiver to direct attention toward a patient rather than a medical device, there is an inherent difficulty in using such systems in most locations, such as, e.g., a nursery, where loud and high pitched cries of infants can cause interference with attempted oral communication between the caregiver and medical device.

Accordingly, it would be advantageous to have a system capable of sending commands to a medical device by means of a caregiver's voice, without requiring the caregiver to physically move towards the device and/or touch the device, yet which commands can be separated from other interference and/or sounds within a given room, ideally without distracting the caregiver from the primary responsibility of caring for the patient.

### Summary

The present system is particularly well-suited for use with maternal and infant care devices. However, it can also be used with numerous other medical devices used to support and/or monitor a patient or the patient's environment. Nevertheless, some of the inventive arrangements will be particularly described as used in a labor and delivery room and/or neonatal intensive care units (NICU), wherein infant cry, for example, is often commonplace and unavoidable. Oftentimes, infant cry comprises a high pitch, loud sound, and it makes voice recognition at a medical device, such as a monitor for example, difficult. As such, the present system provides a remote controller that is separated from a particular medical device that is being controlled, such that the remote control device can be held in the hand of the caregiver.

The system allows for hands-free operation to provide certain commands to the medical device, such that the caregiver can concentrate on the primary responsibility of caring for the patient, such as an infant. There is, therefore, no need to interrupt or otherwise discontinue caring for the patient in order to access a knob or button or the like on the medical device.

The caregiver's voice commands are received by the remote control device, and those signals are then processed by the remote control device and transmitted to the patient device. By such an arrangement, the voice commands are free from other interference within the room, such as the cries of an infant in a nursery, and the command signals are reliably transmitted and received by the patient device for controlling same.

In addition, by utilizing a remote control device that can be held by a caregiver, commands can be spoken into the remote control device in a low and/or muted voice, including a whisper, so as to not disturb the patients being attended to in the room, and which is particularly advantageous, for example, when infants or other patients are sleeping.

As such, with the inventive arrangements, processing the voice of the caregiver occurs in the remote control device, which includes a voice wave receiver, such as a microphone, that receives the caregiver's commands. Such a system can include a speech analyzer that analyzes the speech and recognizes the voice as one that is authorized to give commands to the patient device. If, therefore, for example, an unauthorized user (or other ambient noise and/or the like) attempts to enter voice commands into the remote control device, those voice commands are not recognized and are not accepted into the system as having the authority to command the medical device.

Preferably, the remote control device also includes a command assignment function that receives the voice commands after the commands have been recognized as coming from an authorized caregiver and recognizes a particular command that the caregiver desires to enter into the patient device, such as an oral command of "alarm silence." Such a command can then be converted into digital signals and sent wirelessly to an appropriate patient device.

In one exemplary embodiment, for example, a preferred wireless protocol is the ZigBee protocol, which can be particularly advantageous with the inventive arrangements due to its low power requirements. With other popular protocols, on the other hand, such as Bluetooth, many devices currently use that technology for wireless transmission, and thus, the signals can be subject to interference from other devices. Accordingly, the ZigBee protocol is preferred, although other wireless protocol systems, such as WMTS (Wireless Medical Telemetry System), can also be used with the same advantages as the ZigBee protocol.

The wirelessly transmitted signals represent voice commands and are preferably received by a receiving module at the medical device, which may or may not be physically incorporated thereinto. When not incorporated into the patient device, the receiving module can be a stand alone device that can be placed in communication with the patient device and/or retrofitted into other existing medical patient devices.

As a further option, the remote control device may contain a recording medium as well so that the caregiver can record, for example, the first sounds of a newborn infant or a commentary on the status of the infant or patient for later downloading and/or subsequent review/playback.

These and other features and advantages of the inventive arrangements will become readily apparent from the following detailed description, particularly when taken in conjunction with the drawings herewithin.

### Brief Description of the Drawings

FIG. 1 is a schematic view of a system comprising the inventive arrangements;
FIG. 2 is a schematic view of the remote control device of the inventive arrangements of FIG. 1; and
FIG. 3 is a schematic view of the receiving module of the medical device of the inventive arrangements of FIG. 1.

### Detailed Description of the Inventive Arrangements

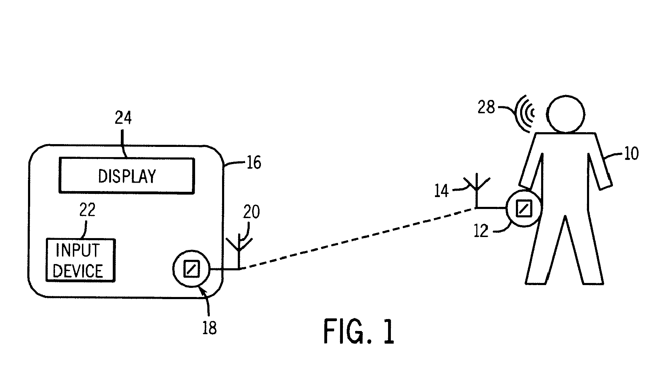

Referring now to FIG. 1, there is shown a schematic view of the system of the inventive arrangements, showing a caregiver 10 caring for a patient (not shown). In the exemplary embodiment, the patient may be a birthing mother, located, for example, in a labor and delivery room, or an infant located in a nursery or NICU unit. As explained, the inventive arrangements are particularly well-suited for use in maternal and/or infant care environments and will be hereafter described as such. However, it will also be seen that the inventive arrangements can also be used in other environments where caregivers and/or the like attend to patients and/or the like.

In any event, in the exemplary environment, the caregiver 10 operates a remote control device 12 that, as will be later explained, is configured to transmit digital signals by a wireless protocol via an antenna 14 to a patient device 16 having a receiving module 18 communicating therewith via an antenna 20 to receive such digital signals. The receiving module 18 receives and interprets the digital signals to carry out some function at the patient device 16 in accordance with the inventive arrangements.

The patient device 16 can be any of a variety of monitors that provide continuous or periodic monitoring of a patient, for example, a birthing mother or a newborn infant, and it conventionally includes a receiving input device 22, such as a knob or keyboard, to input alarm settings, parameters, and/or other information into the patient device 16. The patient device 16 also conventionally includes a display 24, where certain parameters or sensed functions can be displayed for the caregiver 10. The receiving module 18 can be physically incorporated into the patient device 16, or in communication therewith and in general close proximity therewith, such as a receiving module 18 that can be separated from the patient device 16 and/or retrofitted thereto.

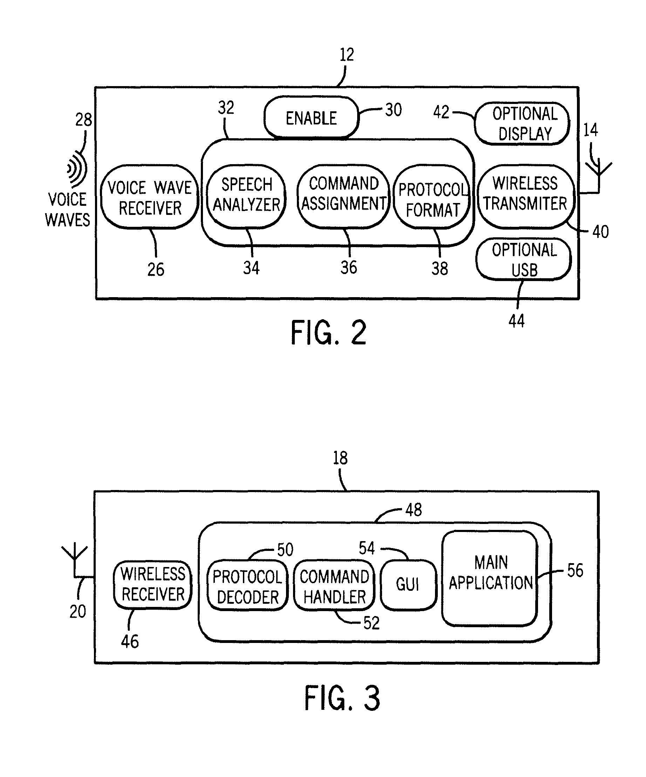

Referring now to FIG. 2, taken along with FIG. 1, there is shown a schematic view of the remote control device 12 used in carrying out the inventive arrangements. More specifically, the remote control device 12 includes a voice wave receiver 26, such as, e.g., a microphone, which receives voice waves 28 from the caregiver 10 in initiating the present system. In the exemplary embodiment, these voice waves 28 carry a command to the patient device 16 to be executed thereby. Typical commands by a caregiver 10 may include, for example, silencing an alarm or altering a setting of the patient device 16, and such functionality would otherwise be carried out by a physical action at the patient device 16, likely through the input device 22 and/or display 24 thereof, thereby removing and/or distracting the caregiver 10 from the primary responsibility of caring for the patient.

Preferably, the remote control device 12 also includes an enable function 30 that allows the caregiver 10 to enable and disable the remote control device 12, that is to say, the caregiver 10 may, by operating the enable function 30, either activate or inactivate the function of the remote control device 12. As such, and as will be seen, when the enable function 30 is not activated, the remote control device 12 will not accept and process commands from the caregiver 10, such that the caregiver 10 only enables the remote control device 12 when the caregiver 10 desires to give a command thereto.

Preferably, the remote control device 12 also includes a software module 32 that contains software that can carry out these inventive arrangements. For example, a speech analyzer 34 may be programmed to recognize a particular caregiver's 10 voice, so that the remote control device 12 knows that a command is being given by an authorized caregiver 10 and not someone that otherwise lacks authority to operate the system. As such, if a particular voice is not recognized by the speech analyzer 34, then the remote control device 12 does not accept or respond to the command.

If, on the other hand, the speech analyzer 34 recognizes the particular caregiver's 10 voice, then the particular command issued by the caregiver 10 is analyzed by a command assignment 36, whereby the command is recognized as requesting a specified action of the patient device 16. The command can also be pre-recorded on the patient device 16 so that the software module 32 can recognize a particular command and execute same by providing an appropriate instruction to the patient device 16. Preferably, the recognized command is then also formatted by a protocol format 38, whereby the software module 32 converts the command into a digital signal by some wireless protocol. In the exemplary embodiment, for example, a preferred wireless protocol is the ZigBee protocol, which is particularly advantageous for the present application since it is a low cost, low power standard, which advantageously allows, for example, longer life with smaller and/or fewer batteries and utilizes a mesh network to enable sufficient reliability and ranges. Other wireless protocols and/or technology can also be used, such as Bluetooth and/or WMTS (Wireless Medical Telemetry System).

Preferably, the actual software utilized in the software module 32 can be conventional, commercially available software, and the software for the speech analyzer 34 and command assignment 36 can be written by one of ordinary skill in the art of software creation without difficulty and/or as a routine matter.

Preferably, the command, now in a wireless format, is transmitted by a wireless transmitter 40 via the antenna 14 of the remote control device 12. Preferably, the transmission of digital signals in accordance with the wireless protocol is not affected by sound interference, such as by the crying of infants and/or the like.

Other features may also be present on the remote control device 12, such as, for example, an optional display 42 that enables the caregiver 10 to read certain functions, such as the status of the remote control device 12, and/or a USB (Universal Serial Bus) input port 44 that can allow the caregiver 10 to interface with other devices, such as a mouse, keyboard, PDA, and/or the like, so that the caregiver 10 can manually input instructions into the remote control device 12 as an option to using voice commands.

Referring now to FIG. 3, taken along with FIGS. 1 and 2, there is shown a schematic view of the receiving module 18 of the patient device 16, such as a patient monitor, that is conventionally present in a room where a patient is located and which monitors a condition of the patient, such as a birthing mother and/or newborn infant. As can be seen, the receiving module 18 preferably includes a wireless receiver 46 to receive the digital signals, as detected by an antenna 20 of the receiving module 18, and those signals are provided to another software module 48. In this instance, the software module 48 preferably includes a protocol decoder 50, whereby the received digital signals can be decoded from the wireless protocol and into decoded signals having the command from the caregiver 10 incorporated thereinto.

Preferably, those signals are analyzed by a command handler 52, which determines and extracts a particular command recognized thereby. Preferably, the command, now recognized, proceeds through a GUI (Graphical User Interface) 54 that interfaces between the command and a main application 56 of the patient device 16, whereby the command can be carried out by the patient device 16 as originally commanded by the voice of the caregiver 10.

As previously stated, the receiving module 18 can be physically incorporated into the patient device 16, or it may also comprise a separate device that can be connected thereto, so as to communicate with the patient device 16. Preferably, the receiving module 18 includes all of the components needed to receive digital signals, decode the protocol, execute commands, and interface with the main application 56 of the patient device 16. As such, in one exemplary embodiment, the receiving module 18 can be a stand alone device that is connected so as to communicate with the patient device 16 and can be retrofitted to existing patient devices currently used in patient care facilities.

Those skilled in the art will recognize that numerous adaptations and modifications can be made to the inventive arrangements for communicating commands to the patient device 16, yet still will fall within the spirit and scope hereof, particularly as defined in the following claims. Accordingly, the inventive arrangements are limited only by the following claims and/or their equivalents.

Aspects of the present invention are defined in the following numbered clauses:
1. A remote control device for receiving and transmitting voice commands for controlling a medical device, comprising:
   a voice recognition system configured to recognize a voice of a particular user and convert commands from the recognized voice into digital signals that represent the command; and
   a transmission system configured to transmit the digital signal by a wireless protocol to a medical device having a receiving module configured to receive the digital signals and control the medical device based on the received digital signals, whereby the medical device is controlled in accordance with the commands of the user.
2. The remote control device of Clause 1, wherein the wireless protocol is a ZigBee wireless protocol.
3. The remote control device of Clause 1 or Clause 2, further comprising:
   an enable control configured to allow input of the commands into the remote control device.
4. The remote control device of any one of the preceding Clauses, further comprising:
   a voice wave receiver configured to receive the voice of the user.
5. The remote control device of any one of the preceding Clauses, further comprising:
   a speech analyzer configured to recognize the voice of the user.
6. The remote control device of any one of the preceding Clauses, further comprising:
   a command assignment configured to recognize particular commands.
7. The remote control device of any one of the preceding Clauses, further comprising:
   a protocol format configured to convert the commands into the digital signals.
8. The system of any one of the preceding Clauses, wherein the medical device is a maternal or infant care device.
9. A voice recognition system for controlling a medical device, comprising:
   a remote control device having i) a voice recognition system configured to recognize a voice of a particular user and convert commands from the recognized voice into digital signals that represent the command, and ii) a transmission system configured to transmit the digital signal by a wireless protocol;
   a medical device having a receiving module configured to receive the digital signals from the remote control device and control the medical device based on the received digital signals, whereby the medical device is controlled in accordance with the commands of the user.
10. The system of Clause 9, wherein the wireless protocol is a ZigBee wireless protocol.
11. The system of Clause 9 or Clause 10, wherein the remote control device includes an enable control configured to allow input of the commands into the remote control device.
12. The system of any one of Clauses 9 to 11, wherein the remote control device includes a voice wave receiver configured to receive the voice of the user.
13. The system of any one of Clauses 9 to 12, wherein the remote control device includes a speech analyzer configured to recognize the voice of the user.
14. The system of any one of Clauses 9 to 13, wherein the remote control device includes a command assignment configured to recognize particular commands.
15. The system of any one of Clauses 9 to 14, wherein the remote control device includes a protocol format configured to convert the commands into the digital signals.
16. The system of any one of Clauses 9 to 15, wherein the medical device is a maternal or infant care device.
17. The system of any one of Clauses 9 to 16, wherein the receiving module is incorporated into the medical device.
18. A voice recognition method for controlling a medical device, comprising:
   receiving a voice command from a user;
   analyzing the voice command to identify the user;
   recognizing the command when the user has been identified as having authority to issue the command;
   converting the recognized command into a digital signal;
   transmitting the digital signal to a medical device by a wireless protocol; and
   executing the command by the medical device.
19. The method of Clause 18, wherein the wireless protocol is a ZigBee wireless protocol.
20. The method of Clause 18 or Clause 19, wherein transmitting the digital signal comprises transmitting the digital signal to a receiving module of the medical device.

## Claims

1. A voice recognition system for controlling a medical device (16), comprising:
a remote control device (12) having i) a voice recognition system configured to recognize a voice of a particular user (10) and convert commands from the recognized voice into digital signals that represent the command, and ii) a transmission system configured to transmit the digital signal by a wireless protocol;
a medical device (16) having a receiving module (18) configured to receive the digital signals from the remote control (12) device and control the medical device (16) based on the received digital signals, whereby the medical device (16) is controlled in accordance with the commands of the user.

2. The system of Claim 1, wherein the wireless protocol is a ZigBee wireless protocol.

3. The system of Claim 1 or Claim 2, wherein the remote control device (12) includes an enable control (30) configured to allow input of the commands into the remote control device (12).

4. The system of any one of the preceding Claims, wherein the remote control device (12) includes a voice wave receiver (26) configured to receive the voice of the user (10).

5. The system of any one of the preceding Claims, wherein the remote control device (12) includes a speech analyzer (34) configured to recognize the voice of the user (10).

6. The system of any one of the preceding Claims, wherein the remote control device (12) includes a command assignment (36) configured to recognize particular commands.

7. The system of any one of the preceding Claims, wherein the remote control device (12) includes a protocol format (28) configured to convert the commands into the digital signals.

8. The system of any one of the preceding Claims, wherein the medical device (16) is a maternal or infant care device.

9. The system of any one of the preceding Claims, wherein the receiving module (18) is incorporated into the medical device (16).

10. A voice recognition method for controlling a medical device (16), comprising:
receiving a voice command from a user (10);
analyzing the voice command to identify the user (10);
recognizing the command when the user (10) has been identified as having authority to issue the command;
converting the recognized command into a digital signal;
transmitting the digital signal to a medical device (16) by a wireless protocol; and
executing the command by the medical device (16).

11. The method of Claim 10, wherein the wireless protocol is a ZigBee wireless protocol.

12. The method of Claim 10 or Claim 11, wherein transmitting the digital signal comprises transmitting the digital signal to a receiving module of the medical device.

13. A remote control device for receiving and transmitting voice commands for controlling a medical device, comprising:
a voice recognition system configured to recognize a voice of a particular user and convert commands from the recognized voice into digital signals that represent the command; and
a transmission system configured to transmit the digital signal by a wireless protocol to a medical device having a receiving module configured to receive the digital signals and control the medical device based on the received digital signals, whereby the medical device is controlled in accordance with the commands of the user.

14. The remote control device of Claim 13, wherein the wireless protocol is a ZigBee wireless protocol.

15. The remote control device of Claim 13 or Claim 14, further comprising:
an enable control configured to allow input of the commands into the remote control device.
